# EUROPEAN PATENT SPECIFICATION

(11) **EP 1 713 328 B1**
(45) Date of publication and mention of the grant of the patent: **09.04.2008**
(21) Application number: 05708324.8
(22) Date of filing: 14.02.2005
(51) Int. Cl.: A01N 25/18, A01N 65/00, A01N 27/00, A61L 2/20, A62D 3/00

(54) **DEACTIVATING EMULSIONS**
DEAKTIVIERENDE EMULSIONEN
EMULSIONS DE DESACTIVATION

(30) Priority: 13.02.2004 GB 0403232
(43) Date of publication of application: 25.10.2006
(73) Proprietor: Reckitt Benckiser (UK) Limited, Slough, Berkshire SL1 3UH (GB); UNIVERSITY OF SOUTHAMPTON, Highfield, Southampton SO17 1BJ (GB)
(72) Inventor: HIGGINS, Sabrina, University of Southampton, Highfield, Southampton SO17 1BJ (GB); HUGHES, John, University of Southampton, Highfield, Southampton SO17 1BJ (GB); McKECHNIE, Malcolm T., Reckitt Benckiser Corp., Hull HU8 7DS (GB)
(74) Representative: Bowers, Craig Malcolm
(86) International application number: PCT/GB2005/000502
(87) International publication number: WO 2005/079571

(56) References cited:
- EP-A- 0 682 942
- EP-A- 0 716 143
- EP-A- 0 843 965
- WO-A-01/76371
- WO-A-93/15774
- WO-A-99/15208
- CA-A1- 2 222 911
- GB-A- 2 367 243
- US-A- 5 085 208
- US-A- 5 271 773
- "Cold Pressed Orange Oil" MATERIAL SAFETY DATA SHEET, [Online] January 2003 (2003-01), XP002332822 Retrieved from the Internet: URL:http://www.pdmchemicals.com/MSDS/MSDS- Orange%20Oil.pdf> [retrieved on 2005-06-20]
- S.BUDAVARI (ED.): "The Merck Index, Eleventh Edition" 1989, MERCK & CO., INC. , XP002332823 entry 7415: "beta-Pinene"
- DATABASE WPI Section Ch, Week 200367 Derwent Publications Ltd., London, GB; Class D16, AN 2003-701561 XP002332824 & JP 2003 180865 A (MITSUBISHI JUKOGYO KK) 2 July 2003 (2003-07-02)

## Description

The present invention relates to a method of deactivating dust mite allergens.

Various allergens are known to trigger a human reaction. For example, it has been known for a long time that house dust can trigger allergenic reactions in humans, such as asthma and rhinitis. It was reported, as early as 1928 that it was the dust mites in the dust that were the primary source of the allergenic response, but it was only in the 1960's that researchers appreciated its significance.

House dust mites produce detritus which causes allergenic reaction in many people. The major allergens are believed to be detritus from the mite species Dermatophogoides farinae and Dermatophagoides pteronyssinus (the allergens being known as Der f1 and Der p1 respectively) The detritus includes faeces as well as body part residues of the mites. A review is given in Experimental and Applied Acarology, 10 (1991) p. 167-186.

WO99/15208 describes a method for deactivating allergens derived from the D. Pteronyssinus and D. Farinae dust mite species, which comprises contacting the allergen with one of 28 deactivants which are described. These may be delivered into an airspace by aerosol spraying.

WO 01/76371 describes further deactivants for house dust mite allergens. These may be delivered into an airspace by various methods including by use of heat to vaporise an oil, an ultra-sonic jet nebuliser, an ion wind, or a candle incorporating a deactivant. In the case of the oil, this may be used as such or may be floated on water or may be presented in the form of an oil-in-water emulsion, generally having up to 5% by weight of the oil.

In accordance with a first aspect of the present invention there is provided a method of deactivating an allergen from the mite species Der f1 or Der p1, the method comprising dispersing into an airspace an allergen-deactivating amount of an allergen-deactivating compound (hereinafter the "deactivant"), the deactivant being provided in the form of an oil-in-water emulsion comprising at least 8% and up to 25% of a deactivant (wt. deactivant/wt. emulsion), and being dispersed into the airspace as a vapour.

Preferably the emulsion comprises at least 9%, and most preferably at least 10%, of a deactivant.

Preferably the deactivant used in the method of the present invention is provided in the form of an oil-in-water emulsion comprising up to 20% of deactivant, preferably up to 18% and most preferably up to 15% of deactivant.

An especially preferred oil-in-water emulsion for use in the method of the present invention comprises 12% of a deactivant.

The percentages given in the above definitions denote the total deactivant content, when there is more than one deactivant present.

In this specification unless otherwise stated a percentage value given for a component denotes the weight of the component expressed as a percentage of the total weight of the emulsion.

Use of the noun deactivant and the verb deactivate in this specification denote that some or all of a source of allergens at a locus are rendered unable to evoke an allergenic response in a human, by a method of the present invention. The net result is that the source may be reduced in its allergenicity, or its allergenicity may be completely removed.

Preferably the deactivant is selected from:
a terpene hydrocarbon;
a citrus oil;
a mint oil;
bois de rose oil;
oil of jasmine;
frankincense;
oil of bergamot; and
oil of lemon grass.

Preferred terpene hydrocarbons include tea tree oil, pinol and β-pinene.

An especially preferred deactivant is a citrus oil, most preferably orange oil.

Another especially preferred deactivant is β-pinene.

A deactivant may suitably be a single compound. Alternatively a mixture of deactivants may be used together.

A deactivant may be part of a blend of compounds, not all of which are deactivants. For example a citrus oil is a blend of compounds not all of which will function as deactivants.

A deactivant may suitably be dispersed into the airspace over an extended period, for example at least 30 minutes, and preferably at least 1 hour.

A deactivant may suitably be dispersed into the airspace on two occasions, interrupted by a period in which there is no deactivant dispersal. A deactivant may be dispersed into the airspace on one or more further occasions, following a corresponding period or periods of no deactivant dispersal. Preferably each such dispersal occasion involves deactivant dispersal over an extended period, as described above. Preferably the or each period in which there is no deactivant dispersal is an extended period, for example at least 2 hours, preferably at least 4 hours, and most preferably at least 8 hours.

We have found that the method produces a prolonged reduction in the allergen loading of an allergen-contaminated inanimate substrate. Delivery of the deactivant into an airspace as described causes a permanent reduction in the population of allergens in an inanimate test source. By inanimate test source we mean a test source which is itself inanimate (e.g. it is not the skin or coat/fur of a live animal) and it does not contain living organisms, such as dust mites. Populations of dust mites would make any result difficult to interpret.

We have found that the reduction in allergen content in such a source is of long duration, for example at least 7 days, typically at least 14 days, and suitably at least 28 days. Indeed, in tests we have carried out over a 28-day period, we have found that the allergen content may continue to decline over time, even though the deactivant may have been used days or weeks before. The results suggest that the allergenic species have been truly denatured or degraded, to the extent that, firstly, they cannot re-form, and secondly, their degradation products are not themselves allergenic. It further suggests that the action of the deactivant is not merely a masking or damping effects. Any such effect would be likely to break down over time.

The formation of emulsions is generally well known in the art and is described, for example, in Modern Aspects of Emulsion Science, edited by Bernard P. Binks, The Royal Society of Chemistry, 1998 and Surfactant Science and Technology, Second Edition, Drew Myers, 1992, VCH Publishers, Inc. Non-ionic surfactants may be especially suitable. Proprietary surfactant packs may be employed to form emulsions, for example E-Z-MULSE (Trade Mark), a non-ionic surfactant pack from Florida Chemical Company, US.

Preferably the deactivant is dispersed into the airspace as a vapour.

Preferably the dispersal of the deactivant is aided by heat applied to the emulsion.

A heat source is preferably located beneath a source of the emulsion. This may, for example, be an oil burner, candle or an electrical heat source, such as a hot plate. Preferably it is a hot plate, preferably having a temperature of at least 100°C.

The use of a hotplate enables the heat applied to vaporise the deactivant to be controlled, in a manner which is not possible with prior methods.

Our work suggests that use of a hotplate below 100°C gives some allergen deactivating activity but that use of a higher temperature gives allergen deactivating activity of a substantially and surprisingly higher level, even though the quantity of deactivant dispersed may be the same in each case.

Preferably the hotplate has an electrical heat source.

Preferably the vessel and the hotplate are in face-to-face contact. Preferably the hotplate has a flat surface and the vessel has a flat base, and the vessel rests on the hotplate. Preferably the vessel has an opening in its upper region. Preferably it has a fully open upper face.

Preferably, therefore, the vessel has a flat base, a side (if cylindrical) or sides depending upwardly therefrom, and nc further side.

Preferably the hotplate is at a temperature of at least 130°C.

Preferably the hotplate is at a temperature up to 300°C, preferably up to 250°C.

The present invention involves the dispersal of an allergen deactivant into an airspace. It is possible that airborne allergens may be deactivated but it is believed that there is effective deactivation of allergens borne on surfaces within the airspace.

In accordance with a further aspect of the present invention there is provided the use of an oil-in-water emulsion in deactivating a Der f1 or Der p1 allergen at a locus, the emulsion comprising a Der f1 or Der p1 allergen deactivant present in a concentration of 10-15% wt./wt. of emulsion, a heat source being used to accelerate the vaporization of the deactivant into an airspace comprising said locus.

The allergen deactivated in a method or use in accordance with the present invention is an allergen from the mite species Der f1 or Der p1 which evokes an allergenic reaction in a human, an allergen arising from house dust mites. The method or use of this invention is able to deactivate, partially or wholly, an allergen arising from the mite species Dermatophogoides farinae (known as Der f1) or, especially from the mite species Dermatophagoides pteronyssinus (known as Der p1).

The present invention will be further described with reference to the following Examples.

### Experimental Protocol

When using house dust for allergen denaturing tests an inherent difficulty is the variability of the amount of allergen in each small sample, even when taken from the same dust reservoir. The amount of dust in the pre-treatment sample must be accurately estimated in order to determine the extent of any allergen denaturing. In these tests the dust sample was applied to the test exposure surface and then one half of this surface dust was removed to measure the control pre-treatment allergen level of that specific sample. Each control was directly relevant to each sample, which gave the best possible estimate of the level of allergen in the sample before exposure to possible denaturant. All tests employed a glass reinforced plastic booth of size 0.7m x 0.7m x 1.0m. Average values are stated.

The following Examples all measure the reduction of the allergen Der p1 from the house dust mite Dermatophagoides pteronyssinus.

House dust was passed through a number of sieves and the fraction smaller than 53 µm was collected. 0.025g of dust was placed in a small sieve to distribute it evenly over the test surface. The test surface was a PTFE (polytetrafluoroethylene - registered trade mark TEFLON) coated metal tray of size 30cm x 30cm. The dust was applied to the tray by moving the sieve continuously over the surface while tapping the sieve. One half of the dust was then removed by suction onto an in-line filter and the weight recorded, this was the pre-treatment control.

The tray was then placed in the booth. Three tea light holders - upwardly open cylindrical vessels (diameter 40mm, height 15mm) produced to hold nightlight candles - each containing 6ml of water and 0.8ml of orange oil - were placed together on an electric hotplate set to 250°C. In practice we found that this meant that the hotplate temperature cycled between 130°C and 250°C. The booth was sealed. Heat was delivered for the period specified below, and then the hotplate was allowed to cool. After 24 hours the tray was removed, the dust was collected from it and its weight recorded. The booth was washed with strong detergent between tests.

Identical tests were carried out differing only in their test liquids. These were:
5% orange oil floated on water (evaporated in 29 minutes) - comparative
12% orange oil floated on water (evaporated in 30 minutes) - comparative
20% orange oil floated on water (evaporated in 20 minutes) - comparative
50% orange oil floated on water (evaporated in 20 minutes) - comparative
12% orange oil/water emulsified with E-Z-MULSE - of the invention (heating stopped after 105 minutes; did not evaporate to dryness. This is believed to be due to remaining non-volatile surfactant from E-Z-MULSE constituent).

The test samples were assayed for the Der p1 allergen using an ELISA (Enzyme linked immunosorbent assay) to determine the allergen content. This was then related to the weight of dust that had been present in each sample. All of the samples were multiplied up to compare the amount of allergen expected to be present in a 0.1g sample of dust. The percentage difference between the control sample and the exposed sample was then obtained.

The Der p1 allergen reductions were as follows:
5% orange oil-on-water - 11.9%
12% orange oil-on-water - 75.4%
20% orange oil-on-water - 67.0%
50% orange oil-on-water - 68.1%
12% orange oil emulsion - 91.0%

The non-volatility of the surfactant content of the emulsion suggested that the orange oil, in emulsion form, was responsible for the activity increase, not the surfactant content itself.

It was found that the allergen content did not substantially recover over time.

Statistical analysis suggested that the increase in activity, from the 12% oil-on-water test liquid to the 12% emulsion, was a significant result.

## Claims

1. A method of deactivating an allergen from the mite species Der f1 or Der p1, the method comprising dispersing into an airspace an allergen-deactivating amount of an allergen-deactivating compound (hereinafter the "deactivant"), the deactivant being provided in the form of an oil-in-water emulsion comprising at least 8% and up to 25% of a deactivant (wt. deactivant/wt. emulsion), and being dispersed into the airspace as a vapour.

2. A method as claimed in claim 1, wherein the deactivant is dispersed into the airspace over a period of at least 30 minutes.

3. A method as claimed in claim 1 or 2, wherein the dispersal is aided by heat applied to the emulsion.

4. A method as claimed in any preceding claim, wherein the deactivant is selected from:
a terpene hydrocarbon;
a citrus oil;
a mint oil;
bois de rose oil;
oil of jasmine;
frankincense;
oil of bergamot;
oil of lemon grass;
or a component thereof.

5. A method as claimed in any preceding claim, wherein the deactivant comprises a terpene hydrocarbon.

6. A method as claimed in any preceding claim, wherein the deactivant comprises β-pinene.

7. A method as claimed in any preceding claim, wherein the deactivant comprises orange oil or a component thereof.

8. The use of an oil-in-water emulsion in deactivating a Der f1 or Der p1 allergen at a locus, the emulsion comprising a Der f1 or Der p1 allergen deactivant present in a concentration of 10-15% wt./wt. of emulsion, a heat source being used to accelerate the vaporization of the deactivant into an airspace comprising said locus.

## Patentansprüche

1. Verfahren zum Deaktivieren eines Allergens der Milbenspezies Der-fl oder Der-p1, wobei das Verfahren das Verteilen einer allergendeaktivierenden Menge einer allergendeaktivierenden Verbindung (hier nachstehend das "Deaktivierungsmittel") in einem Luftraum, wobei das Deaktivierungsmittel in Form einer Öl-in-Wasser-Emulsion bereitgestellt ist, die mindestens 8% und bis zu 25% eines Deaktibvierungsmittels (Gew. Deaktivierungsmittel/Gew. Emulsion) umfasst, und im Luftraum als Dampf verteilt wird.

2. Verfahren nach Anspruch 1, wobei das Deaktivierungsmittel über eine Dauer von mindestens 30 Minuten im Luftraum verteilt wird.

3. Verfahren nach Anspruch 1 oder 2, wobei die Verteilung durch auf die Emulsion aufgebrachte Wärme unterstützt wird.

4. Verfahren nach einem der vorangehenden Ansprüche, wobei das Deaktivierungsmittel ausgewählt ist aus
einem Terpen-Kohlenwasserstoff;
einem Zitrusöl;
einem Minzöl;
Rosenholzöl;
Jasminöl;
Weihrauch;
Bergamottöl;
Zitronengrasöl;
oder einem Bestandteil davon.

5. Verfahren nach einem der vorangehenden Ansprüche, wobei das Deaktivierungsmittel einen Terpen-Kohlenwasserstoff umfasst.

6. Verfahren nach einem der vorangehenden Ansprüche, wobei das Deaktivierungsmittel β-Pinen umfasst.

7. Verfahren nach einem der vorangehenden Ansprüche, wobei das Deaktivierungsmittel Orangenöl oder einen Bestandteil davon umfasst.

8. Verwendung einer Öl-in-Wasser-Emulsion beim Deaktivieren eines Der-f1-oder Der-p1-Allergens an einem Ort, wobei die Emulsion eine Der-d1-oder Der-pl-Allergendeaktivierungsmittel umfasst, das in einer Konzentration von 10-15 Gew.-% der Emulsion vorliegt, wobei eine Wärmequelle zum Beschleunigen der Verdampfung des Deaktivierungsmittels in den den Ort umfassenden Luftraum verwendet wird.

## Revendications

1. Procédé pour désactiver un allergène de l'espèce d'acarien Der f1 ou Der p1, procédé comprenant la dispersion dans un espace d'air d'une quantité, désactivatrice d'allergène, d'un composé désactivateur d'allergène (ci-après le "désactivant"), le désactivant étant fourni sous forme d'une émulsion huile-dans-eau comprenant au moins 8 % et jusqu'à 25 % d'un désactivant (poids de désactivant/poids d'émulsion), et étant dispersé dans l'espace d'air sous forme d'une vapeur.

2. Procédé suivant la revendication 1, dans lequel le désactivant est dispersé dans l'espace d'air en une période de temps d'au moins 30 minutes.

3. Procédé suivant la revendication 1 ou 2, dans lequel la dispersion est facilitée par de la chaleur appliquée à l'émulsion.

4. Procédé suivant l'une quelconque des revendications précédentes, dans lequel le désactivant est choisi entre :
un hydrocarbure terpénique ;
une essence d'agrume ;
une essence de menthe ;
une essence de bois de rose ;
une essence de jasmin ;
l'essence d'encens ;
l'essence de bergamote ;
l'essence de lemongrass ;
ou un de leurs constituants.

5. Procédé suivant l'une quelconque des revendications précédentes, dans lequel le désactivant comprend un hydrocarbure terpénique.

6. Procédé suivant l'une quelconque des revendications précédentes, dans lequel le désactivant comprend le β-pinène.

7. Procédé suivant l'une quelconque des revendications précédentes, dans lequel le désactivant comprend l'essence d'orange ou un de ses constituants.

8. Utilisation d'une émulsion huile-dans-eau dans la désactivation d'un allergène de Der f1 ou Der p1 dans un lieu, l'émulsion comprenant un désactivant d'allergène de Der f1 ou Der p1 présent en une concentration de 10 à 15 % en poids/poids de l'émulsion, une source de chaleur étant utilisée pour accélérer la vaporisation du désactivant dans un espace d'air comprenant ledit lieu.
